# EUROPEAN PATENT APPLICATION

(11) **EP 0 859 010 A1**
(43) Date of publication of application: **19.08.1998**
(21) Application number: 96900448.0
(22) Date of filing: 16.01.1996
(51) Int. Cl.: C07K 16/24, C12P 21/08, C12N 15/06, G01N 33/577

(54) **ANTI-TPO MONOCLONAL ANTIBODY**

(30) Priority: 17.01.1995 JP 49/95
(71) Applicant: Kirin Brewery Company, Ltd., Tokyo 104 (JP)
(72) Inventor: MIYAZAKI, Hiroshi, Kirin Brewery Company, Limited, Maabashi-shi, Gunma-ken 371 (JP)
(74) Representative: UEXKÜLL & STOLBERG
(86) International application number: JP9600050
(87) International publication number: WO9622309

(57) **Abstract**

The present invention relates to a monoclonal antibody to human thrombopoietin (TPO), to a method for the purification of TPO which comprises isolating TPO from a TPO-containing material by affinity chromatography using the antibody, and to a method for the immunochemical quantification or detection of TPO using the antibody. Various types of human TPO molecules can be purified, detected or quantified by use of the anti-TPO monoclonal antibody.

## Description

### BACKGROUND OF THE INVENTION

### Field of the Invention:

This invention relates to an anti-TPO monoclonal antibody useful for detection, purification or quantification of TPO.

### Disclosure of Related Art:

Human TPO (thrombopoietin) is a protein cloned as ligand of Mpl which is a member of the cytokine receptor superfamily (de Sauvage et al., Nature (London), vol.369, pp.533-565 (1994); Bartley, T.D. et al., Cell, vol.77, pp.1117-1124 (1994)). Each of these Mp1 ligands can be detected in sera and blood plasmas of thrombocytopenic animals (human, mouse, dog), and its involvement in the production of megakaryocytes and platelets has been already confirmed.

To develop a therapeutic agent for use in the treatment of thrombocytopenia, the present inventors have purified rat TPO from plasma of thrombocytopenic rats by using, as an indication, an activity that stimulates the production of megakaryocytes from megakaryocytic precursor cells highly purified from rat bone marrow, and have succeeded in cloning rat TPO cDNA and human TPO cDNA based on its partial amino acid sequence and in obtaining homogeneous human TPO in a large quantity by the recombinant DNA technology (H. Miyazaki et al., Exp. Hematol., vol.22, p.838 (1994)). It has been found that the resulting human TPO has the same amino acid sequence as that of the above mentioned factor obtained as human Mp1 ligand (see SEQ ID NO:1 in SEQUENCE LISTING set forth below).

In this context, superior means for the detection, purification or quantification of TPO are needed for production of homogeneous human TPO in a large quantity by the recombinant DNA technology, for evaluation of the TPO prepared, or for development of the TPO as drugs.

### SUMMARY OF THE INVENTION

The present inventors have studied on preparation of anti-TPO monoclonal antibodies useful for the detection, purification or quantification of TPO, and found that hybridomas obtained by fusion of myeloma cells with antibody-producing cells derived from an animal which has been immunized with a human TPO mutant (referred to as "h6T(1-163)") expressed in *Escherichia coli,* produced monoclonal antibodies which can recognize h6T(1-163), a human TPO protein that has the amino acid sequence shown in SEQ ID NO:1 and is expressed in CHO cells (referred to as "CHO(1-332)"), an other human TPO protein which has an amino acid sequence of the positions 1 through 163 shown in SEQ ID NO:1 and is expressed in CHO cells (referred to as "CHO(1-163)"), etc., and that these monoclonal antibodies could be used for purification and quantification of the human TPO proteins.

The h6T(1-163) has an amino acid sequence (SEQ ID NO:2) which corresponds to the positions 1 through 163 of the amino acid sequence shown in SEQ ID NO:1, except that the amino acid residues Ser¹ and Ala³ are substituted by Ala and Val, respectively, and that Met and Lys residues are additionally attached to the N-terminus. A process for the production of h6T(1-163) will be illustrated in detail in Example 1 set forth below.

Accordingly, the present invention provides a monoclonal antibody to human TPO. It also provides a method for the purification of TPO which comprises isolating the TPO from a TPO-containing material by affinity chromatography using an anti-human TPO monoclonal antibody. The present invention further provides a method for an immunochemical quantification or detection of TPO which comprises the use of an anti-human TPO monoclonal antibody.

### BRIEF DESCRIPTION OF THE DRAWINGS

Fig. 1 is a graph showing change in the 450/570 nm absorbance depending on the dose of CHO(1-163) or CHO(1-332).

Fig. 2 is a graph showing change in the 450/570 nm absorbance depending on the content (or concentration) of CHO(1-163), CHO(1-332), MKT(L-163) or MKT (1-332).

Fig. 3 is a graph showing reactivities of the monoclonal antibody of the present invention with CHO(1-332) and recombinant human erythropoietin, which are compared by absorbance ratio of A450/A570.

### DETAILED DESCRIPTION OF THE INVENTION

The present invention will be described below in more detail.

The monoclonal antibody of the present invention can be produced in the following manner.

### (1) Preparation of immunized animal cells

A mouse, rat or the like is immunized with human TPO, from which spleen cells are prepared. For example, the human TPO can be produced in *E*. *coli* by using recombinant DNA techniques (see Example 1). Immunization is generally carried out by administering 5 to 100 µg of human TPO/animal in combination with an appropriate adjuvant into a mouse, rat or rabbit, subcutaneously, intravenously or intraperitoneally, or in a manner of foot-pad administration. The immunization may be carried out once or several times at appropriate intervals, preferably at intervals of 1 to 2 weeks (in the case of foot-pad administration, 2 to 3 days).

Antibody titers against antigens in sera of the immunized animals are measured and, based on this results, animals that showed a sufficiently high antibody titer are used as the source of antibody producing cells. Preferred are antibody producing cells obtained 3-4 days after the final immunization.

Various known techniques such as radioimmunoassay (RIA), enzyme-linked immunosorbent assay (ELISA) and the like can be used as methods for measurement of an antibody titer, and ELISA is normally used in this invention.

### (2) Preparation of myeloma cells

As the myeloma cells, it is desirable in general to use established cells obtained from mice, for example, 8-azaguanine-resistant mouse (BALB/c origin) myeloma cell lines such as P3-X63 Ag8-U1 (P3-U1) [Current Topics in Microbiology and Immunology, 81, 1-7 (1978)], P3-NSI/1-Ag4.1 (NS-1) [European J. Immunology, 6, 511-519 (1976)), SP2/O-Ag14 (SP-2) (Nature, 276, 269-270 (1978)], P3-X63-Ag8.653 (653) (J. Immunology, 123, 1548-1550 (1979)], P3-X63-Ag8 (X63) [Nature, 256, 495 - 497 (1975)] and the like. These cell lines are subcultured in or on an appropriate medium such as 8-azaguanine medium [8-azaguanine plus the nomal medium i.e. RPMI 1640 medium supplemented with glutamine (1.5 mM), 2-mercaptoethanol (5 x 10⁻⁵ M), gentamicin (10 µg/ml) and fetal calf serum (FCS; 10%); IMDM (Iscove Modified Dulbecco's Medium); or Dulbecco's MEM, and they are finally subcultured in or on the normal medium 3 to 4 days before the cell fusion is carried out so that 2 x 10⁷ or more fusion cells can be obtained at the time of fusion.

### (3) Cell fusion

In the case of mouse, for example, 5 to 100 µg of human TPO/animal is administered intraperitoneally or subcutaneously into mice that has been immunized in the manner of step (1) and, after 3-4 days, the spleens of the mice are removed in order to obtain spleen cells. The spleen cells thus obtained are well washed with a culture medium or PBS as well as myeloma cells prepared in step (2), after which both cells are mixed in a ratio of spleen cells to myeloma cells, about 5-10:1, and then subjected to centrifugation. The resulting supernatant is discarded, and the precipitated cells are thoroughly loosened and stirred at 37°C for 1 to 2 minutes while adding a solution of polyethylene glycol (PEG: molecular weight, 1,000 to 4,000) in a medium like DMEM to the suspension until the number of spleen cells becomes 10⁸ cells per 0.1-1.0 ml of the suspension, and the stirring is continued for additional 1 to 2 minutes. After stirring, 1 to 3 ml of the medium is added to the suspension while stirring for 1 to 3 minutes and subsequently adding 7 to 10 ml of the same medium slowly. The suspension is left for 5 to 10 minutes at 37°C and then centrifuged. The resulting supernatant is discarded and the remaining cells are resuspended in FCS-supplemented medium and subjected to centrifugation. This procedure is repeated twice or three times. The cells are gently suspended in 50 to 200 ml of FCS-supplemented medium, and the suspension is dispensed in an amount of half the well plume into wells of a culture plate and cultured at 35 to 38°C for 10 to 18 hours using a 3-7% CO₂ incubator. HAT medium (hypoxanthine (10⁻⁶ to 10⁻³ M), aminopterin (10⁻⁸ to 10⁻⁷ M) and thymidine (10⁻⁶ to 10⁻⁴ M) in the normal medium) is dispensed in an amount of half the well volume into the wells of the culture plate, and the cultivation is continued over 10 to 30 hours. Thereafter, at intervals of 10 to 30 hours over 1 to 4 days a half volume of the culture supernatant fluid is removed and replaced by the same volume of the fresh HAT medium, and the cells are cultured at 35 to 38°C over 10 to 14 days using a 3-7% CO₂ incubator.

When hybridoma cells grown in the shape of a colony are observed in a well, a half volume of the supernatant fluid in the well is removed and replaced by the same volume of an HT medium (aminopterin-free HAT medium), and the substitution of the supernatant fluid by HT medium is subsequently carried out at intervals of 10 to 30 hours over 1 to 3 days. After 3 to 4 days of cultivation in HT medium, a portion of the culture supernatant is taken from the culture and measured for anti-TPO antibody titer by the enzyme immunoassay.

A hybridoma whose productivity of a specific antibody was confirmed by the above antibody titer measurement is transferred to an other plate to carry out its cloning. This cloning may be performed by a method in which the hybridoma is diluted by limiting dilution so that a single hybridoma cell is contained in one well, a soft agar method in which a colony is isolated from a soft agar medium, a method in which a single hybridoma cell is picked up using a micromanipulator, or a "sorter clone" method in which a single hybridoma cell is separated using a cell sorter, and preferred is the limiting dilution method which is simple or easy for handling. As to wells in which the antibody titer was confirmed, the cloning was repeated 2 to 4 times, for example, by the limiting dilution method, and clones showing a stable antibody titer are selected as anti-TPO antibody producing hybridoma cell lines.

### (4) Preparation of monoclonal antibody

The hybridomas obtained in the above step (3) are cultured in the normal medium in place of the HT medium. A large scale culture is carried out using spinner or roller bottles. An anti-human TPO monoclonal antibody can be obtained, for example, by subjecting the supernatant fluid obtained from the large scale culture to a gel filtration to collect and purify an IgG fraction.

Alternatively, when the immunized animal is a mouse, the hybridomas can be grown in the abdominal cavities of other mice belonging to similar lines (e.g., Balb/c or Nu/Nu). In this case, when the anti-TPO monoclonal antibody producing hybridomas obtained in the above step (3) are administered into pristane-treated female mice (4 to 8 weeks of age) by intraperitoneal injection in a dose of 4 x 10⁴-10⁷ cells/animal, the hybridomas cause ascites tumor 2 to 3 weeks after the administration. When the ascitic fluid is collected from each of the mice and any solid components are removed by centrifugation, it can be used as a monoclonal antibody for the detection or quantification and purification of human TPO. If further purification is required, IgG fraction may be collected from the centrifuged supernatant using a DEAE-Sepharose column, a Protein A-Sepharose column or the like.

Determination of an isotype or subclass of the antibody is carried out by the Ouchterlony method (Introduction to immunological Experiments, Methods for Biochemical Experiments 15, published by Gakkai Shuppan Center, Tokyo, Japan, p.74, 1981) or EIA.

The protein content is calculated by the Folin-Lowry method and by the absorbance at 280 nm [1.4(OD₂₈₀) = immunoglobulin 1 mg/ml].

Methods for the preparation of the monoclonal antibodies of the present invention will be described in the following Examples in which monoclonal antibodies (designated as L3-1 and L4-1) obtained from the hybridoma cell lines L3-1 and L4-1 were found to belong to subclasses IgG 2a and IgG 2b, respectively.

Antigen specificities of the obtained monoclonal antibodies L3-1 and L4-1 are described in the following Examples.

The monoclonal antibodies of the present invention can be used as ligands in an affinity chromatography for the isolation and purification of human TPO. Immobilized monoclonal antibodies useful in the affinity chromatography can be prepared according to various protein immobilization techniques using a gel support such as CNBr-activated Sepharose 4B (Pharmacia Fine Chemicals) or a membrane support. For purification of human TPO using the immobilized monoclonal antibody, the antibody is packed in a column and a solution containing human TPO is applied thereto. Thereafter, unbound substances are washed out and the TPO bound to the column is eluted, for example, using glycine-HCl buffer (pH 2.5) that may contain sodium chloride, propionic acid, dioxane, ethylene glycol, chaotropic salt, gluanidine hydrochloride, urea or the like.

The monoclonal antibodies of the present invention can be used in the determination of human TPO, for example, by an enzyme immunoassay such as solid phase sandwich method.

According to an embodiment of the present invention, the detection or quantification method of the present invention comprises contacting the anti-TPO monoclonal antibody with a TPO-containing sample to detect or quantify a formed immunological complex.

The solid phase sandwich method is carried out by the following procedure.

Solid phase sandwich method is a type of noncompetitive solid phase enzyme immunoassays in which an antigen is detected or quantified with two different antibodies which can recognize different epitopes of the same antigen. That is, an antibody which recognizes TPO is immobilized on a solid phase in a first step, after which, in a second step, the TPO is adsorbed onto the solid phase through formation of an immune complex of TPO with the antibody immobilized on the solid phase. In a third step, the TPO is detected or quantified through its reaction with a labeled antibody or antibody fragment thereof that can recognize a TPO epitope other than the epitope recognizable by the antibody used in the first step. The labeled antibody or its fragment can be prepared by labeling it with an enzyme such as horseradish peroxidase directly by the maleimide-hinge method or the like, or indirectly by the avidin-biotin method or the like.

### EXAMPLES

The following examples are provided to illustrate the present invention in more detail.

### Example 1 Preparation of an antigenic protein, h6T(I-163)

DNA coding for the h6T(1-163) shown in SEQUENCE LISTING (SEQ ID NO:3) was chemically synthesized and cloned into pCFM536 (ATCC No. 39934; JP-A-60-501988) which has been digested with XbaI and HindIII [E. coli JM109 transformed in advance with pMWl (ATCC No.39933) was used as the host]. The thus obtained clone was named pCFM536/h6T(1-163), and the *E*. *coli* strain containing this expression vector was used as a transformant for use in the expression of h6T(1-163).

Expression of the expression plasmid pCFM536 is controlled by a λPL promoter which is under control of a cI857 repressor gene. The transformant described above was cultured overnight at 30°C with shaking in 60 ml of LB medium supplemented with 50 µg/ml of ampicillin and 12.5 µ/ml of tetracycline, and 25 ml of the culture was added to 1,000 ml of LB medium supplemented with 50 µg/ml of ampicillin and cultured at 30°C with shaking until OD at A*600* reached 1.0 to 1.2. Subsequently, about 330 ml of LB medium prewarmed to 65°C was added so that the final temperature became 42°C, and the shaking culture was continued for 3 hours at 42°C to induce expression of h6T(1-163).

0.6 g of the frozen cells of the h6T(1-163) producing transformant were suspended in 3 ml of water and disrupted using a high pressure disintegrater. A precipitated fraction was recovered by centrifugation, thereby removing most of the contaminated proteins, cell components and the like. The thus recovered precipitated fraction containing h6T(1-163) was suspended in 2 ml of water, mixed with 0.5 ml of 1 M Tris-HCl buffer pH 8.5 and 10 ml of 10 M urea and then stirred at room temperature for 5 minutes. To 4 ml of the resulting suspension were added 28 ml of 20 mM Tris-HCl buffer pH 8.5 and 8 ml of 20 mM Tris-HCl buffer pH 8.5 containing 1 M urea, followed by overnight gentle stirring at 4°C. A precipitated fraction was recovered by centrifugation, and the thus recovered precipitated fraction containing h6T(1-163) was solubilized at room temperature by adding 200 µl of 0.5 M Tris-HCl buffer pH 8.5 containing 8 M guanidine, 5% acetonitrile and 0.3% EDTA and then subjected to 1 hour of reduction treatment at 60°C after addition of 20 mg of DTT. Next, using an elution solvent A (0.1% TFA) and an elution solvent B (n-PrOH containing 0.05% TFA), a 100 µl portion of this was applied to a Capcell Pak C1 300A (manufactured by Shiseido, Catalog (Ca.) No. C1 TYPE:SG300A, ⌀4.6 x 150 mm) column equilibrated with 25% B, and then developed at a flow rate of 0.4 ml/min with 50 minutes of a linear gradient of from 25% B to 40% B to recover a peak fraction of h6T(1-163) eluted within the range from about 28% to about 30% n-PrOH. After addition of 100 µl of 50% glycerol to this fraction, TFA and n-PrOH were removed by centrifugation-evaporation to prepare h6T(1-163) which was used as the antigen.

### Example 2 Preparation of hybridomas producing anti-human TPO monoclonal antibodies

### (1) Immunization of mice

500 µg of h6T(1-163) obtained in Example 1 was well mixed with 1,000 µl of RIVI ADJUVANT (manufactured by IMMUNOCHEM RESEARCH, Ca.No. 52017700) to form an emulsion. Next, 5 µg/head of the antigen was injected into the foot pad of each Balb/c female mouse (4 weeks of age) under etherization. Boosters were repeated 8 times in total in the same manner as above at intervals of 3 days. At the time of the final booster, human IL-6 was administered by subcutaneous injection at a dose of 5 µg/head. After 3 days of the final booster, blood was collected from the orbit using a hematocrit capillary tube, and serum was separated therefrom to measure blood antibody titer and reactivity with h6T(1-163) by means of an enzyme immunoassay method. Spleen cells of mice which showed 1:1000 or more of the antibody titer and TPO reactivity were used in subsequent cell fusion.

### (2) Preparation of myeloma cell line

Myeloma cells (P3X63-Ag·8-653) were thawed, suspended in 10% FCS (manufactured by Hyclone, Ca.No. A-1115-L)-added DMEM medium and then centrifuged at 300 x g for 5 minutes. The supernatant was discarded, and the remaining cells were resuspended in 10% FCS/DMEM and cultured at 37°C in a 5% CO₂ incubator.

### (3) Cell fusion

Cell fusion was carried out in the usual way. Briefly, the regional (i.e., popliteal, inguinal and mesenteric) lymph nodes and the spleen of a mouse immunized with h6T(1-163) were aseptically removed, and the lymph node cells and spleen cells were separately suspended in 10% FCS/DMEM to prepare antibody producing cells. Next, 1 part of myeloma cells at logarithmic growth phase were mixed with 5 parts of the lymph node cells or spleen cells, washed with DMEM to remove serum and then subjected to cell fusion using polyethylene glycol (PEG 1500, manufactured by Boehringer Mannheim, Ca. No. 783641). After completion of the cell fusion, the resulting cells were adjusted to 1 x 10⁶ cells/ml with DMEM containing 10% FCS and 10 ng/ml human IL-6 and dispensed in 100 µl portions into wells of a 96-well microtiter plate (Nunc-Immuno Plate MaxiSorp™, manufactured by InterMed, Ca.No. 4-42404). After 18 hours, 10% FCS/10 ng/ml-human IL-6/DMEM containing 2 x HAT (manufactured by SIGMA, Ca.No. H0262) was added to the wells in an amount of 100 µl/well, and the cells were cultured over about 10 to about 14 days at 37°C in a 5% CO₂ incubator.

### (4) Screening of hybridomas producing anti-human TPO monoclonal antibodies

Hybridomas producing anti-human TPO monoclonal antibody were screened by an enzyme immunoassay method. The h6T(1-163) or CHO(1-332) was adjusted to 20 µg/ml with PBS and dispensed in 50 µl portions into wells of a 96-well microtiter plate (Nunc-Immuno Plate MaxiSorp™, manufactured by InterMed, Ca.No. 4-42404). The antigen was thoroughly spread on the bottom of each well while shaking the plate using a microplate mixer (MICROPLATE MIXER MPM-1, manufactured by Iwaki Glass). The plate was sealed with a plate seal (manufactured by SUMILON, Ca.No. MS-30020) and left at 37°C for 2 hours or at 4°C overnight to adsorb the antigen onto the plate. Next, after washing with a washing buffer of 20 mM Tris-HCl/0.5 M NaCl/0.1% Tween 20 (pH 7.5), 300 µl of 4-fold diluted Block Ace (manufactured by Dainippon Pharmaceuticals, Ca.No. UK-B25) was added to each well, and the plate was sealed with the plate seal and left at 37°C for 1 hour or at 4°C overnight. After 4 times of washing with the washing buffer, 50 µl of culture supernatant fluid of a hybridoma clone was added to each well, and the plate was shaken using a microplate mixer, sealed with the plate seal and then subjected to 1 hour of reaction at 37°C. After completion of the reaction and subsequent 4 times of washing with the washing buffer, 50 µl of alkaline phosphatase-labeled Protein A (PIERCE, Ca.No. 32300) which had been diluted 1:500 in 10-fold diluted Block Ace was added to each well, and the plate was shaken on a microplate mixer, sealed with the plate seal and then subjected to 1 hour of reaction at 37°C. After the reaction, the wells were washed four times with the washing buffer, 100 µl of a color former of a color developing kit for alkaline phosphatase (Kirkegaard & Perry Laboratories, Ca.No. 50-80-00) was added to each well, and the plate was shaken on a microplate mixer, sealed with the plate seal and then subjected to the reaction at room temperature. After about 30 minutes, the absorbance at 405 nm was measured using a plate reader (Wellreader SK601, manufactured by Seikagaku Kogyo). Clones positive for both h6T(1-163) and CHO(1-332) were selected from the wells. The thus selected clones were expanded and frozen until use. At the same time, cloning was carried out by the limiting dilution method. The limiting dilution was carried out using 10% FCS/10 ng/ml-human IL-6/DMEM medium containing 1 x HT (manufactured by SIGMA, Ca.No. H0137), and a second screening was carried out in the same manner as above by the enzyme immunoassay method. When the clones were stabilized, they were subcultured in 10% FCS/DMEM, expanded, and then frozen until use.

### (5) Recognition site of anti-human TPO monoclonal antibodies

By the above method, a total of 63 anti-human TPO monoclonal antibody clones were obtained. From these clones, 13 clones having high reactivity were selected, and their regions that react with a TPO protein were determined by the same enzyme immunoassay method employed in the above step (4), using multiple antigen peptides (HT-1, 2, 3, 4, 5 and 6) (see Table 1) which had been synthesized based on the human TPO amino acid sequence shown in SEQ ID NO:1. As the results, one antibody which reacts with HT-1 (antibody L3-1), one antibody which reacts with HT-2 (antibody L4-1) and 10 antibodies which react with HT-3 (antibodies L1-10, S6-27, S3-13, S2-2, L3-15, L2-43, L3-4, L4-13, S4-12 and S3-52) were obtained.

**Table 1**

| |
|---|
| Human TPO (amino acid positions 8 to 28) peptide HT-1 region DLRVLSKLLRDSHVLHSRLSQ |
| Human TPO (amino acid positions 47 to 62) peptide HT-2 region SLGEWKTQMEETKAQD |
| Human TPO (amino acid positions 108 to 126) peptide HT-3 region LGTQLPPQGRTTAHKDPNA |
| Human TPO (amino acid positions 172 to 190) peptide HT-4 region NELPNRTSGLLETNFTASA |
| Human TPO (amino acid positions 262 to 284) peptide HT-5 region SLPPNLQPGYSPSPTHPPTGQYT |
| Human TPO (amino acid positions 306 to 332) peptide HT-6 region PSAPTPTPTSPLLNTSYTHSQNLSQEG |
| Depositary authority: National Institute of Bioscience and Human-Technology, Agency of Industrial Science and Technology. Address : 1-3, Higashi 1-chome, Tsukuba-shi, Ibaraki-ken, Japan. Depositary authority: China Center for Type Culture Collection. Address : Luo Jia Shan, Wuhan 430072, China. |

In this connection, a subclone (L4-1-31) of the hybridoma L4-1 and a subclone (L3-1-54) of the hybridoma L3-1, have been deposited on December 27, 1994 with the National Institute of Bioscience and Human-Technology, Agency of Industrial Science and Technology, Ministry of International Trade and Industry, Japan, under Accession Nos. FERM BP-4956 and FERM BP-4955, respectively. These subclones have been also deposited with the Chinese depositary authority (CCTCC) under Accession Nos. CCTCC-C95003 and CCTCC-C95002, the subclones being named Mouse-Mouse hybridoma L4-1-31 and Mouse-Mouse hybridoma L3-1-54, respectively.

### (6) Subclass of anti-human TPO monoclonal antibodies

Subclass of the anti-human TPO monoclonal antibodies L3-1, L4-1, L1-10, S6-27 and S3-13 obtained in the above step (5) was determined by the EIA method (INNOGENETICS). Each culture supernatant was reacted with an immobilized rat anti-mouse (subclass specific) monoclonal antibody, followed by the reaction with an alkaline phosphatase-labeled rat anti-mouse (κ/light chain specific) monoclonal antibody. After completion of the reaction, color development was performed by adding BCIP substrate to determine a subclass. As the results, subclasses of the anti-human TPO monoclonal antibodies L3-1, L4-1, L1-10, S6-27 and S3-13 were found to be IgG2a, IgG2b, IgG3, IgG3 and IgG3, respectively.

### (7) Purification of anti-human TPO monoclonal antibodies

The hybridomas producing anti-human TPO monoclonal antibodies were cultured in one liter of a serum-free eRDF medium supplemented with 10 mg of bovine insulin, 10 mg of human transferrin, 20 mM of monoethanolamine and 5 x 10⁻⁵ M of sodium selenit in a large scale using roller bottles (Falco, Ca.No. 3000). When the survival of the hybridoma reached 90% or below, centrifugation was carried out to recover culture supernatant which was subsequently filtered through a 0.22 µm membrane filter. The resulting culture supernatant was applied at a flow rate of 100 ml/min, to a Protein A column (manufactured by Fuji Filter Industries K.K., Japan) which had been equilibrated with a 0.15 M phosphate buffer (PBS). After adsorption of the antibody in the culture supernatant onto the Protein A, non-adsorbed substances were washed out by passing PBS through the column at a flow rate of 100 ml/min until the absorption value at 280 nm reached the base line. After the non-adsorbed substances were washed out, the antibody was eluted and recovered with 0.1 M glycine eluent (pH 2.5). The recovered solution was neutralized with 0.1 M Tris, adjusted to an appropriate concentration by ultrafiltration, sterilized and then stored at -20°C.

### Example 3 ELISA using anti-human TPO monoclonal antibodies

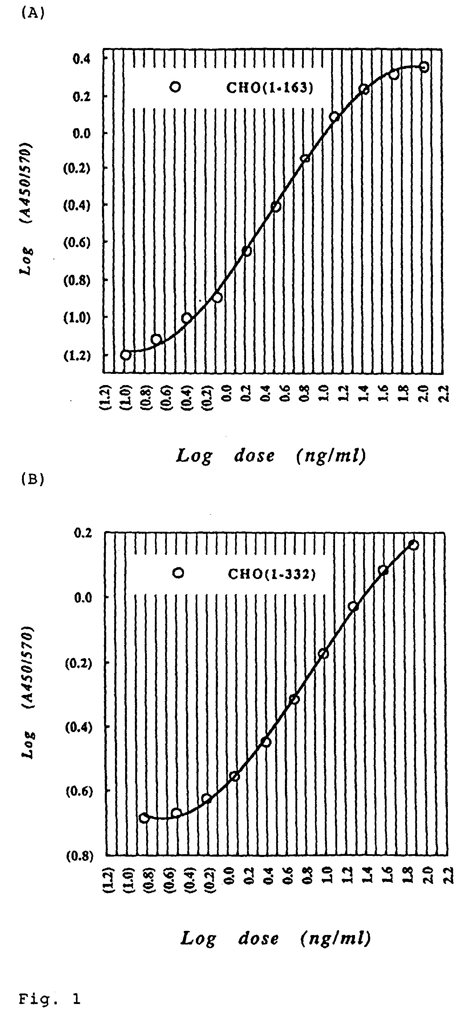

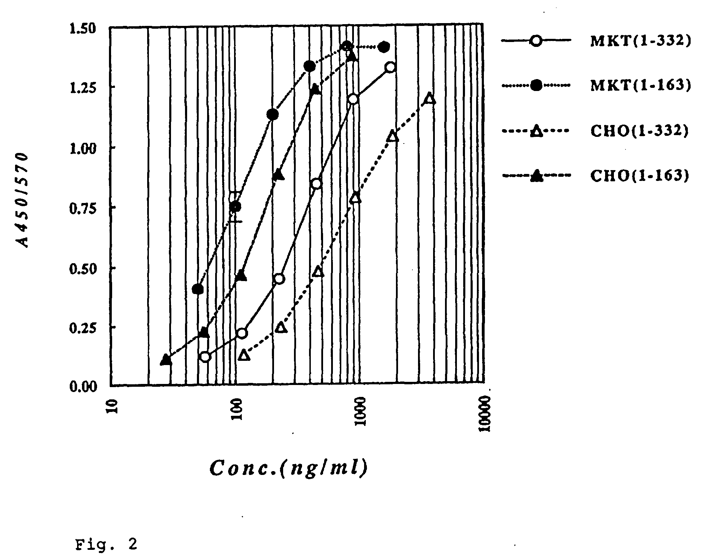

An anti-human TPO monoclonal antibody (subclone L3-1-54 of L3-1) which recognizes the HT-1 region as a solid phase antibody was adjusted to 20 µg/ml with 50 mM carbonate buffer (pH 9.2) and dispensed in 50 µl portions into wells of a 96-well microtiter plate (Nunc-Immuno Plate MaxiSorp™, manufactured by InterMed, Ca.No. 4-42404). The solid phase antibody was thoroughly spread on the bottom of each well by shaking the plate using a microplate mixer (MIKROPLATE MIXER MPM-1, manufactured by Iwaki Glass). The plate was sealed with a plate seal (manufactured by SUMILON, Ca.No. MS-30020) and left at 37°C for 2 hours or at 4°C overnight to adsorb the solid phase antibody onto the plate. After washing with a washing buffer (20 mM Tris-HC1/0.5 M NaCl/0.1% Tween 20 (pH 7.5)), 300 µl of 4-fold diluted Block Ace (Dainippon Pharmaceuticals, Ca.No. UK-B25) was added to each well, and the plate was sealed with a plate seal and left at 37°C for 1 hour or at 4°C overnight. After 4 times of washing with the washing buffer, 50 µ1 of a sample to be tested or a known concentration of TPO as a standard diluted in 10-fold diluted Block Ace was added to each well, and the plate was shaken on the microplate mixer, sealed with a plate seal and then left at 37°C for 2 hours or at 4°C overnight. After the reaction, the wells were washed four times with the washing buffer, a biotin-labeled anti-human TPO monoclonal antibody (subclone L4-1-31 of L4-1; primary antibody) which recognizes the HT-2 region was diluted to 500 ng/ml with 10-fold diluted Block Ace and dispensed in 50 µl portions into the wells, and the plate was shaken on the microplate mixer, sealed with a plate seal and then left at 37°C for 2 hours or at 4°C overnight. After the reaction, the wells were washed four times with the washing buffer, peroxidase-labeled avidin (UltraAvidin™-Horseradish Peroxidase, manufactured by Leinco Technologies, Ca.No. A106) was diluted 1:2,000 in 10-fold diluted Block Ace and dispensed in 50 µl portions into the wells, and the plate was shaken on a microplate mixer, sealed with a plate seal and then subjected to 1 hour of reaction at 37°C. After completion of the reaction and subsequent washing (x 4) with the washing buffer, 100 µl of a color former prepared by adding 1/100 volumes of a substrate solution to a TMBZ color former of a color developing kit for peroxidase (SUMILON, Ca.No. ML-1120T) was added to each well, and the plate was shaken on the microplate mixer, sealed with a plate seal and then subjected to the reaction at room temperature. After about 20 minutes, 100 µl of a reaction termination solution was added to each well, and the plate was shaken on a microplate mixer to stop the coloring reaction. Absorbances at wave lengths of 450 nm/570 nm were measured using an ELISA plate reader (Wellreader SK601, manufactured by Seikagaku Kogyo). A standard curve was made based on absorption values obtained by known concentrations of CHO(1-163) or CHO(1-332) (see Fig. 1). In addition to the CHO(1-163) and CHO(1-332), another standard curve was also made for human TPO mutants produced in *E*. *coli* (for example, "MKT(1-332)" and "MKT(1-163)") (see Fig. 2). Thus, various types of TPO molecules can be measured quantitatively.

MKT(1-332) has an amino acid sequence in which a Met residue (at the position -2) and a Lys residue (at the position -l) are added to the N-terminus of the human TPO amino acid sequence shown in SEQ ID NO: 1. MKT(1-163) has an amino acid sequence in which a Met residue (at the position -2) and a Lys residue (at the position -1) are added to the N-terminus of the 1-163 amino acid sequence shown in SEQ ID NO:1.

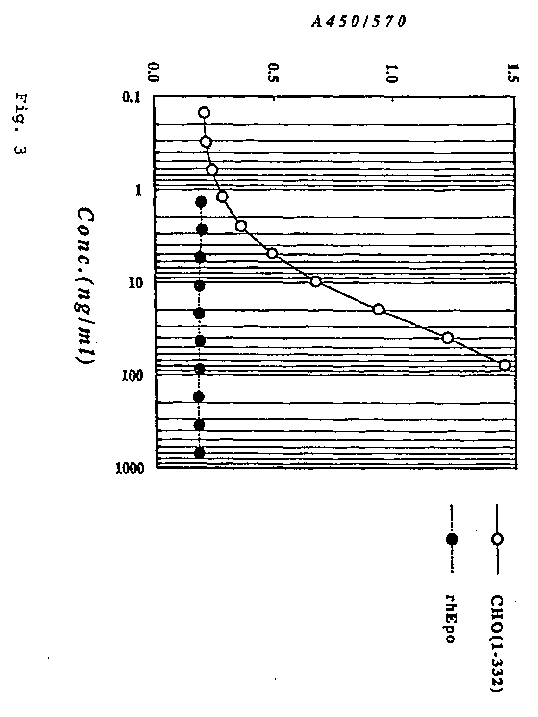

In addition, the antigen specificity was examined using human erythropoietin (hEPO) which shows a high homology with an N-terminal amino acid sequence of human TPO shown in SEQ ID NO:1. As a result, it was found that this system can detect TPO specifically (see Fig. 3).

Also, the detection or quantification of TPO was able to be carried out like the case of the combination of L3-1-54 and L4-1-31, when sandwich assay was carried out using a combination of L3-1 and S3-13, S3-13 and L3-1, or S3-13 and L4-1 as the combination of an solid phase antibody and a biotin-labeled first antibody.

As illustrated in the above specific examples, various types of human TPO molecules can be detected or quantified by use of the monoclonal antibodies of the present invention.

SEQUENCE LISTING

INFORMATION FOR SEQ ID NO:1:
SEQUENCE CHARACTERISTICS:
LENGTH: 332 amino acids
TYPE: amino acid
ORIGINAL SOURCE:
Homo sapiens
SEQUENCE DESCRIPTION: SEQ ID NO:1:
Ser Pro Ala Pro Pro Ala Cys Asp Leu Arg Val Leu Ser Lys Leu Leu
1               5                   10                  15
Arg Asp Ser His Val Leu His Ser Arg Leu Ser Gln Cys Pro Glu Val
            20                  25                  30
His Pro Leu Pro Thr Pro Val Leu Leu Pro Ala Val Asp Phe Ser Leu
        35                  40                  45
Gly Glu Trp Lys Thr Gln Met Glu Glu Thr Lys Ala Gln Asp Ile Leu
    50                  55                  60
Gly Ala Val Thr Leu Leu Leu Glu Gly Val Met Ala Ala Arg Gly Gln
65                  70                  75                  80
Leu Gly Pro Thr Cys Leu Ser Ser Leu Leu Gly Gln Leu Ser Gly Gln
                85                  90                  95
Val Arg Leu Leu Leu Gly Ala Leu Gln Ser Leu Leu Gly Thr Gln Leu
            100                 105                 110
Pro Pro Gln Gly Arg Thr Thr Ala His Lys Asp Pro Asn Ala Ile Phe
        115                 120                 125
Leu Ser Phe Gln His Leu Leu Arg Gly Lys Val Arg Phe Leu Met Leu
    130                 135                 140
Val Gly Gly Ser Thr Leu Cys Val Arg Arg Ala Pro Pro Thr Thr Ala
145                 150                 155                 160
Val Pro Ser Arg Thr Ser Leu Val Leu Thr Leu Asn Glu Leu Pro Asn
                165                 170                 175
Arg Thr Ser Gly Leu Leu Glu Thr Asn Phe Thr Ala Ser Ala Arg Thr
            180                 185                 190
Thr Gly Ser Gly Leu Leu Lys Trp Gln Gln Gly Phe Arg Ala Lys Ile
        195                 200                 205
Pro Gly Leu Leu Asn Gln Thr Ser Arg Ser Leu Asp Gln Ile Pro Gly
    210                 215                 220
Tyr Leu Asn Arg Ile His Glu Leu Leu Asn Gly Thr Arg Gly Leu Phe
225                 230                 235                 240

Pro Gly Pro Ser Arg Arg Thr Leu Gly Ala Pro Asp Ile Ser Ser Gly
                245                 250                 255
Thr Ser Asp Thr Gly Ser Leu Pro Pro Asn Leu Gln Pro Gly Tyr Ser
            260                 265                 270
Pro Ser Pro Thr His Pro Pro Thr Gly Gln Tyr Thr Leu Phe Pro Leu
        275                 280                 285
Pro Pro Thr Leu Pro Thr Pro Val Val Gln Leu His Pro Leu Leu Pro
    290                 295                 300
Asp Pro Ser Ala Pro Thr Pro Thr Pro Thr Ser Pro Leu Leu Asn Thr
305                 310                 315                 320
Ser Tyr Thr His Ser Gln Asn Leu Ser Gln Glu Gly
                325                 330

INFORMATION FOR SEQ ID NO:2:
SEQUENCE CHARACTERISTICS:
          LENGTH: 165 amino acids
          TYPE: amino acid
ORIGINAL SOURCE:
          Homo sapiens
SEQUENCE DESCRIPTION: SEQ ID NO:2:
Met Lys Ala Pro Val Pro Pro Ala Cys Asp Leu Arg Val Leu Ser Lys
-2  -1   1               5                  10
Leu Leu Arg Asp Ser His Val Leu His Ser Arg Leu Ser Gln Cys Pro
15                  20                  25                  30
Glu Val His Pro Leu Pro Thr Pro Val Leu Leu Pro Ala Val Asp Phe
                35                  40                  45
Ser Leu Gly Glu Trp Lys Thr Gln Met Glu Glu Thr Lys Ala Gln Asp
            50                  55                  60
Ile Leu Gly Ala Val Thr Leu Leu Leu Glu Gly Val Met Ala Ala Arg
        65                  70                  75
Gly Gln Leu Gly Pro Thr Cys Leu Ser Ser Leu Leu Gly Gln Leu Ser
    80                  85                  90
Gly Gln Val Arg Leu Leu Leu Gly Ala Leu Gln Ser Leu Leu Gly Thr
 95                 100                 105                 110
Gln Leu Pro Pro Gln Gly Arg Thr Thr Ala His Lys Asp Pro Asn Ala
                115                 120                 125
Ile Phe Leu Ser Phe Gln His Leu Leu Arg Gly Lys Val Arg Phe Leu
            130                 135                 140
Met Leu Val Gly Gly Ser Thr Leu Cys Val Arg Arg Ala Pro Pro Thr

145 150 155
Thr Ala Val Pro Ser
160 163

INFORMATION FOR SEQ ID NO:3:
SEQUENCE CHARACTERISTICS:
LENGTH: 535 nucleic acids
TYPE: nucleic acid
STRANDEDNESS: double
TOPOLOGY: linear
MOLECULE TYPE: synthetic DNA
ORIGINAL SOURCE:
Homo sapiens
SEQUENCE DESCRIPTION: SEQ ID NO:3:
CTAGAAAAAA CCAAGGAGGT AATAAATA 28
ATG AAA GCA CCT GTA CCA CCT GCA TGT GAT TTA CGG GTC CTG TCT AAA 76
Met Lys Ala Pro Val Pro Pro Ala Cys Asp Leu Arg Val Leu Ser Lys
+1 5 10
CTG CTG CGC GAC TCT CAC GTG CTG CAC TCT CGT CTG TCC CAG TGC CCG 124
Leu Leu Arg Asp Ser His Val Leu His Ser Arg Leu Ser Gln Cys Pro
15 20 25 30
GAA GTT CAC CCG CTG CCG ACC CCG GTT CTG CTT CCG GCT GTC GAC TTC 172
Glu Val His Pro Leu Pro Thr Pro Val Leu Leu Pro Ala Val Asp Phe
35 40 45
TCC CTG GGT GAA TGG AAA ACC CAG ATG GAA GAG ACC AAA GCT CAG GAC 220
Ser Leu Gly Glu Trp Lys Thr Gln Met Glu Glu Thr Lys Ala Gln Asp
50 55 60
ATC CTG GGT GCA GTA ACT CTG CTT CTG GAA GGC GTT ATG GCT GCA CGT 268
Ile Leu Gly Ala Val Thr Leu Leu Leu Glu Gly Val Met Ala Ala Arg
65 70 75
GGC CAG CTT GGC CCG ACC TGC CTG TCT TCC CTG CTT GGC CAG CTG TCT 316
Gly Gln Leu Gly Pro Thr Cys Leu Ser Ser Leu Leu Gly Gln Leu Ser
80 85 90
GGC CAG GTT CGT CTG CTG CTC GGC GCT CTG CAG TCT CTG CTT GGC ACC 364
Gly Gln Val Arg Leu Leu Leu Gly Ala Leu Gln Ser Leu Leu Gly Thr
95 100 105 110
CAG CTG CCG CCA CAG GGC CGT ACC ACT GCT CAC AAG GAT CCG AAC GCT 412
Gln Leu Pro Pro Gln Gly Arg Thr Thr Ala His Lys Asp Pro Asn Ala
115 120 125

ATC TTC CTG TCT TTC CAG CAC CTG CTG CGT GGC AAA GTT CGT TTC CTG 460
Ile Phe Leu Ser Phe Gln His Leu Leu Arg Gly Lys Val Arg Phe Leu
130 135 140
ATG CTG GTT GGC GGT TCT ACC CTG TGC GTT CGT CGG GCG CCG CCA ACC 508
Met Leu Val Gly Gly Ser Thr Leu Cys Val Arg Arg Ala Pro Pro Thr
145 150 155
ACT GCT GTT CCG TCT TAATGAAAGC TT 535
Thr Ala Val Pro Ser
160

## Claims

1. A monoclonal antibody to human TPO.

2. A method for the purification of TPO which comprises isolating TPO from a TPO-containing material by affinity chromatography using a monoclonal antibody to human TPO.

3. A method for immunochemically quantifying or detecting TPO, characterized by the use of a monoclonal antibody to human TPO.

4. The method according to claim 3 which comprises contacting the monoclonal antibody with a TPO-containing sample to quantify or detect a formed immunological complex.
